# EUROPEAN PATENT APPLICATION

(11) **EP 4 226 924 A1**
(43) Date of publication of application: **16.08.2023**
(21) Application number: 21876964.4
(22) Date of filing: 28.09.2021
(51) Int. Cl.: A61K 31/439, A61P 27/10, A61P 27/02

(54) **USE OF PENEHYCLIDINE IN TREATMENT OR PREVENTION OF VISION-IMPAIRING EYE DISEASES**

(30) Priority: 10.10.2020 CN 202011080237; 01.09.2021 CN 202111023964
(71) Applicant: Grand Life Sciences Group (Wuhan) Co., Ltd., Wuhan, Hubei 430040 (CN)
(72) Inventor: HUANG, Ling, Wuhan, Hubei 430040 (CN); ZHANG, Xiaohua, Wuhan, Hubei 430040 (CN); ZHU, Mo, Wuhan, Hubei 430040 (CN); XIANG, Wendian, Wuhan, Hubei 430040 (CN); LIU, Chao, Wuhan, Hubei 430040 (CN); KANG, Jing, Wuhan, Hubei 430040 (CN)
(74) Representative: DehnsGermany Partnerschaft von Patentanwälten
(86) International application number: PCT/CN2021/121385
(87) International publication number: WO 2022/073446

(57) **Abstract**

Provided is a use of a compound represented by Formula (I) or a nitrogen oxide, solvate, metabolite, pharmaceutically acceptable salt, or prodrug thereof in the preparation of medicament.

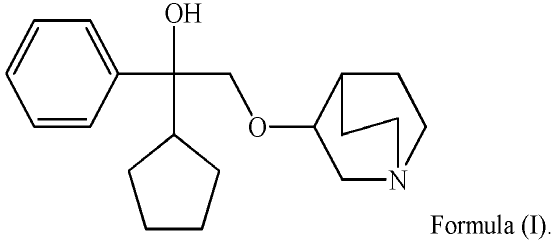

## Description

### TECHNICAL FIELD

The present disclosure relates to the field of medicine. In particular, the present disclosure relates to use of penehyclidine in treatment or prevention of vision-impairing eye diseases.

### BACKGROUND

According to epidemiological reports, the incidence of amblyopia is 1.6% to 3.6% in China, and up to 11.8% in some regions, and the prevalence rate of myopia among the China's population over 5 years old will increase to about 51%, and the affected population will reach 700 million.

Currently, atropine eye drops are recommended for the prevention and treatment of myopia and amblyopia in foreign countries, for example, 1% atropine sulfate eye drops marketed in the United States and Japan, with indications of mydriasis for diagnostic or therapeutic purposes and accommodation paralysis and amblyopia treatment. In recent years, low-concentration atropine sulfate eye drops (0.01%) have been approved in Taiwan and Macao regions for alleviating or treating myopia, and clinical experiments for relieving the progression of myopia have been reported in Hong Kong, Singapore, the United States, UK and India, indicating that low-concentration atropine can effectively delay the progression of myopia in children and adolescents.

Among hypotheses for the pathogenesis of myopia, it has been proposed that myopia progression is related to ciliary muscle accommodation. Sato's regulation theory suggests that long-term regulation may result in regulatory tension of ciliary muscle, and thus it is speculated that relaxation and release of regulatory tension by using atropine may achieve the effect of juvenile myopia control. Atropine can block mammalian ciliary muscle M receptors, thereby alleviating the regulatory tension of ciliary muscle. In contrast, chick ciliary muscle is striated muscle, which is regulated by nicotine (N)-like receptors, and thus atropine cannot paralyze chick ciliary muscle. However, McBrien *et al.* induced -2.8D of myopia with an ocular axis extension of 0.21 mm on day 8 by injecting 0.01% by mass of atropine into the vitreous cavity of chick modeled eyes with visual form deprivation myopia (FDM), while chicks in the shaminjected group and the saline-injected group were induced with -18.5D of myopia with an ocular axis extension of 1.04 mm and -20.9D of myopia with an ocular axis extension of 1.00 mm, respectively. That is, atropine can still significantly inhibit the formation and progression of myopia in chicks, suggesting that atropine inhibits myopia through a non-regulatory mechanism or cycloplegic paralysis is not the only target of atropine myopia control. Atropine has been proven to be capable of preventing the progression of myopia in some animal models and humans. However, the mechanism of the atropine's anti-myopia effect is not fully understood.

At present, the relationship between various medicaments and the treatment of myopia has not been established. Since atropine is a non-selective inhibitor, it has been found that atropine is associated with multiple pathways for the treatment of myopia. Tropicamide is also another non-selective M receptor blocker, which has been proven to be ineffective in preventing or delaying the progression of myopia through the comparison experiments with atropine.

Stone *et al.* reported in 1991 that daily subconjunctival injection of pirenzepine, an M1 receptor blocker, could inhibit the occurrence of FDM, while methoctramine, an M2 receptor blocker, and 4-DAMP, an M3 receptor blocker, were ineffective, suggesting that they may be related to M1 receptors. However, the study of pirenzepine found that the experimental results of different research groups in the treatment of myopia were not consistent. In contrast, Flitcroft applied another M1 receptor blocker, trihexyphenidyl, through topical eye drops or oral administration, which could reach relatively high retinal concentrations, and the results indicate that it is not effective in preventing the progression of experimental myopia.

Luft *et al.* studied more than ten medicaments with M cholinergic receptor-blocking effect, including atropine (non-selective), pirenzepine (M1) and Oxyphenonium (M2), and found that only Oxypheneonium, atropine, pirenzepine and Himbacine had certain effect in preventing FDM without retinal damage, while other medicaments could not prevent FDM or have different degrees of retinal damage. M receptors can mediate various functions, and different subtypes of M receptors in the same tissue have even different pharmacological properties. In the respiratory tract, the function of the M2 receptor is diametrically opposed to that of the M1 and M3 receptors. Therefore, the selectivity for different subtypes of M receptors is the key to determine the application prospect of anticholinergic medicaments. Therefore, the relationship between M receptors and myopia has not been established from the reports in the research literature.

In conclusion, it is urgent to develop new medicaments for treating or preventing visual impairment such as myopia and amblyopia.

So far, there is no report about the effect of penehyclidine and its derivatives in the prevention and treatment of myopia and amblyopia.

### SUMMARY

In the treatment of myopia and amblyopia, atropine sulfate eye drops currently under research abroad have a certain risk. Atropine may easily produce systemic side effects, such as dry mouth, increased heart rate, urinary retention, etc., and even causing poisoning and anaphylactic shock. Intravenous maximum dose is 2mg per time, exceeding such a dose will cause poisoning. Overdose is characterized by clumsy and unstable action, unclear mind, convulsion, dyspnea and abnormal heartbeat, etc. Thus, the safety of atropine sulfate eye drops used in myopia and amblyopia has been a factor limiting its large-scale promotion. At present, it is urgent to find safer and more effective medicaments for the treatment of eye diseases on the market.

The present disclosure aims to solve one of the technical problems in the related art at least to a certain extent.

To this end, in a first aspect of the present disclosure, the present disclosure provides use of a compound having a structure represented by Formula (I), or a nitrogen oxide, solvate, metabolite, pharmaceutically acceptable salt or prodrug thereof, in the preparation of a medicament for treating and/or preventing vision-impairing eye diseases,

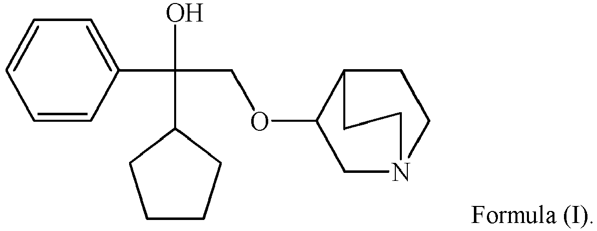

The Applicant has found through a large number of studies that the compound having the structure represented by Formula (I) or its derivative (nitrogen oxide, solvate, metabolite, pharmaceutically acceptable salt) has a good effect in the treatment and/or prevention of vision-impairing eye diseases, and the systemic side effects thereof are significantly weaker than those of atropine, thereby having a good application prospect.

According to an embodiment of the present disclosure, the above-mentioned use may further comprise at least one of the following additional technical features:

According to an embodiment of the present disclosure, the salt of the compound represented by Formula (I) includes organic salts or inorganic salts.

The compound represented by Formula (I) is referred as to penehyclidine. Through a large number of studies, the Applicant has surprisingly and delightfully found that penehyclidine and derivatives thereof, for example, organic salts or inorganic salts thereof, can effectively treat or prevent vision-impairing eye diseases such as myopia or amblyopia, and they have better prevention and control effects than atropine sulfate eye drops of the same concentration and advantages such as smaller mydriatic effect, smaller local eye irritation and better safety. Thus, they are suitable for long-term use.

According to an embodiment of the present disclosure, the salt of the compound represented by Formula (I) is a hydrochloride salt having the structure represented by Formula (II):

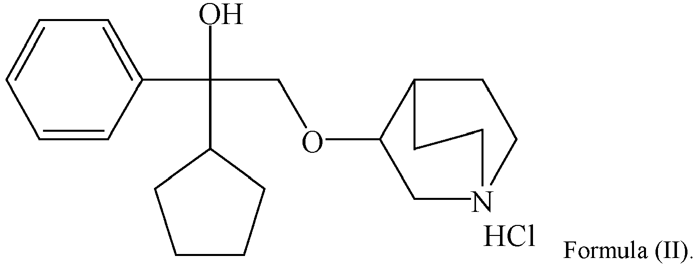

Through repeated experiments, the Applicant has found that the above-mentioned form of penehyclidine hydrochloride has a good effect in the treatment and/or prevention of diseases such as myopia and amblyopia.

According to an embodiment of the present disclosure, the medicament is for use in the treatment and/or prevention of myopia and/or amblyopia.

According to embodiments of the present disclosure, the myopia and/or amblyopia is caused by one or more selected from myopic shift in diopter, myopic vitreous cavity depth extension, myopic axial length extension, visual form deprivation, anisometropia, and astigmatism.

According to an embodiment of the present disclosure, the myopia and/or amblyopia includes one or more of mild myopia, moderate myopia, high myopia, axial myopia, simple myopia, pathological myopia, distance vision loss, asthenopia, exotropia, strabismus amblyopia, fundus damage, visual occlusion, visual distortion, double vision, abnormal color vision, abnormal light vision, decreased contrast sensitivity, and ametropic amblyopia. It should be noted that the vision-impairing eye diseases described herein do not include vision problems caused by trauma.

In a second aspect of the present disclosure, the present disclosure provides a pharmaceutical composition for treating and/or preventing vision-impairing eye diseases. According to an embodiment of the present disclosure, the composition includes, as an active ingredient, the compound having the structure represented by Formula (I), or the nitrogen oxide, solvate, metabolite, pharmaceutically acceptable salt or prodrug thereof.

According to an embodiment of the present disclosure, the above-mentioned pharmaceutical composition may further include at least one of the following additional technical features:

According to an embodiment of the present disclosure, the pharmaceutical composition is for use in the treatment and/or prevention of myopia and/or amblyopia.

According to embodiments of the present disclosure, the myopia and/or amblyopia is caused by one or more selected from myopic shift in diopter, myopic vitreous cavity depth extension, myopic axial length extension, visual form deprivation, anisometropia, and astigmatism.

According to an embodiment of the present disclosure, the myopia and/or amblyopia includes one or more of mild myopia, moderate myopia, high myopia, axial myopia, simple myopia, pathological myopia, distance vision loss, asthenopia, exotropia, strabismus amblyopia, fundus damage, visual occlusion, visual distortion, double vision, abnormal color vision, abnormal light vision, decreased contrast sensitivity, and ametropic amblyopia. It should be noted that the vision-impairing eye diseases described herein do not include vision problems caused by trauma.

According to an embodiment of the present disclosure, the pharmaceutical composition further includes at least one pharmaceutically acceptable carrier. In the pharmaceutical composition according to the embodiments of the present disclosure, the carriers include, but are not limited to, aqueous solvents, water-miscible solvents, non-aqueous solvents, antimicrobial agents or preservatives against the growth of microorganisms, stabilizers, solubility enhancers, osmotic pressure regulators, buffers, antioxidants, local anesthetics, suspending and dispersing agents, wetting or emulsifying agents, complexing agents, sequestering or chelating agents, antifreeze agents, cryoprotectants, thickening agents, pH-adjusting agents, and inert gases, which can be selected by those skilled in the art according to the actual requirements of the formulation.

According to an embodiment of the present disclosure, the pharmaceutical composition is in in a formulation of an ophthalmic preparation, and the ophthalmic preparation includes one or more of eye drops, eye ointment, eye cream, eye emulsion, eye gel, eye pill, eye membrane, or intraocular implant.

According to an embodiment of the present disclosure, a mass fraction of the active ingredient in the pharmaceutical composition ranges from 0.005% to 2%, based on a mass of the pharmaceutical composition. In the pharmaceutical composition according to the embodiments of the present disclosure, the mass volume fraction of the active ingredient in the pharmaceutical composition is 0.005%, 0.01%, 0.015%, 0.02%, 0.025%, 0.03%, 0.035%, 0.04%, 0.045%, 0.05%, 0.055%, 0.06%, 0.065%, 0.07%, 0.075%, 0.08%, 0.085%, 0.09%, 0.095%, 0.1%, 0.105%, 0.11%, 0.115%, 0.12%, 0.125%, 0.13%, 0.135%, 0.14%, 0.145%, 0.15%, 0.155%, 0.16%, 0.165%, 0.17%, 0.175%, 0.18%, 0.185%, 0.19%, 0.195%, 0.2%, 0.205%, 0.21%, 0.215%, 0.22%, 0.225%, 0.23%, 0.235%, 0.24%, 0.245%, 0.25%, 0.255%, 0.26%, 0.265%, 0.27%, 0.275%, 0.28%, 0.285%, 0.29%, 0.295%, 0.3%, 0.305%, 0.31%, 0.315%, 0.32%, 0.325%, 0.33%, 0.335%, 0.34%, 0.345%, 0.35%, 0.355%, 0.36%, 0.365%, 0.37%, 0.375%, 0.38%, 0.385%, 0.39%, 0.395%, 0.4%, 0.405%, 0.41%, 0.415%, 0.42%, 0.425%, 0.43%, 0.435%, 0.44%, 0.445%, 0.45%, 0.455%, 0.46%, 0.465%, 0.47%, 0.475%, 0.48%, 0.485%, 0.49%, 0.495%, 0.5%, 0.505%, 0.510%, 0.515%, 0.520%, 0.525%, 0.530%, 0.535%, 0.540%, 0.545%, 0.550%, 0.555%, 0.560%, 0.565%, 0.570%, 0.575%, 0.580%, 0.585%, 0.590%, 0.595%, 0.600%, 0.605%, 0.610%, 0.615%, 0.620%, 0.625%, 0.630%, 0.635%, 0.640%, 0.645%, 0.650%, 0.655%, 0.660%, 0.665%, 0.670%, 0.675%, 0.680%, 0.685%, 0.690%, 0.695%, 0.700%, 0.705%, 0.710%, 0.715%, 0.720%, 0.725%, 0.730%, 0.735%, 0.740%, 0.745%, 0.750%, 0.755%, 0.760%, 0.765%, 0.770%, 0.775%, 0.780%, 0.785%, 0.790%, 0.795%, 0.800%, 0.805%, 0.810%, 0.815%, 0.820%, 0.825%, 0.830%, 0.835%, 0.840%, 0.845%, 0.850%, 0.855%, 0.860%, 0.865%, 0.870%, 0.875%, 0.880%, 0.885%, 0.890%, 0.895%, 0.900%, 0.905%, 0.910%, 0.915%, 0.920%, 0.925%, 0.930%, 0.935%, 0.940%, 0.945%, 0.950%, 0.955%, 0.960%, 0.965%, 0.970%, 0.975%, 0.980%, 0.985%, 0.990%, 0.995%, 1.000%, 1.005%, 1.010%, 1.015%, 1.020%, 1.025%, 1.030%, 1.035%, 1.040%, 1.045%, 1.050%, 1.055%, 1.060%, 1.065%, 1.070%, 1.075%, 1.080%, 1.085%, 1.090%, 1.095%, 1.100%, 1.105%, 1.110%, 1.115%, 1.120%, 1.125%, 1.130%, 1.135%, 1.140%, 1.145%, 1.150%, 1.155%, 1.160%, 1.165%, 1.170%, 1.175%, 1.180%, 1.185%, 1.190%, 1.195%, 1.200%, 1.205%, 1.210%, 1.215%, 1.220%, 1.225%, 1.230%, 1.235%, 1.240%, 1.245%, 1.250%, 1.255%, 1.260%, 1.265%, 1.270%, 1.275%, 1.280%, 1.285%, 1.290%, 1.295%, 1.300%, 1.305%, 1.310%, 1.315%, 1.320%, 1.325%, 1.330%, 1.335%, 1.340%, 1.345%, 1.350%, 1.355%, 1.360%, 1.365%, 1.370%, 1.375%, 1.380%, 1.385%, 1.390%, 1.395%, 1.400%, 1.405%, 1.410%, 1.415%, 1.420%, 1.425%, 1.430%, 1.435%, 1.440%, 1.445%, 1.450%, 1.455%, 1.460%, 1.465%, 1.470%, 1.475%, 1.480%, 1.485%, 1.490%, 1.495%, 1.500%, 1.505%, 1.510%, 1.515%, 1.520%, 1.525%, 1.530%, 1.535%, 1.540%, 1.545%, 1.550%, 1.555%, 1.560%, 1.565%, 1.570%, 1.575%, 1.580%, 1.585%, 1.590%, 1.595%, 1.600%, 1.605%, 1.610%, 1.615%, 1.620%, 1.625%, 1.630%, 1.635%, 1.640%, 1.645%, 1.650%, 1.655%, 1.660%, 1.665%, 1.670%, 1.675%, 1.680%, 1.685%, 1.690%, 1.695%, 1.700%, 1.705%, 1.710%, 1.715%, 1.720%, 1.725%, 1.730%, 1.735%, 1.740%, 1.745%, 1.750%, 1.755%, 1.760%, 1.765%, 1.770%, 1.775%, 1.780%, 1.785%, 1.790%, 1.795%, 1.800%, 1.805%, 1.810%, 1.815%, 1.820%, 1.825%, 1.830%, 1.835%, 1.840%, 1.845%, 1.850%, 1.855%, 1.860%, 1.865%, 1.870%, 1.875%, 1.880%, 1.885%, 1.890%, 1.895%, 1.900%, 1.905%, 1.910%, 1.915%, 1.920%, 1.925%, 1.930%, 1.935%, 1.940%, 1.945%, 1.950%, 1.955%, 1.960%, 1.965%, 1.970%, 1.975%, 1.980%, 1.985%, 1.990%, 1.995%, or 2.000%.

According to a specific embodiment of the present disclosure, an administration dosage of the pharmaceutical composition depends on the species and weight of the subject to be treated, the nature and severity of disease, the type of formulation, administration route, and the administration period or time interval.

In a third aspect of the present disclosure, the present disclosure provides an ophthalmic preparation for treating and/or preventing vision-impairing eye diseases. According to an embodiment of the present disclosure, a mass fraction of an active ingredient in the ophthalmic preparation ranges from 0.005% to 2%, and the active ingredient is the compound having the structure represented by Formula (I), or the nitrogen oxide, solvate, metabolite, pharmaceutically acceptable salt or prodrug thereof. Through a large number of experiments, the Applicant found that such an ophthalmic preparation has a definite dose-related relaxation effect on ciliary muscle and a significant effect in delaying the ocular axis, and it can delay the progression of myopia. Therefore, the ophthalmic preparation can exert the effect of treating myopia, and allows the amblyopia trend in animal models with amblyopia gradually to recover and the incubation period to be gradually shortened. When the ophthalmic preparation is administered for a certain time, the amblyopia can be basically recovered to normal level.

According to an embodiment of the present disclosure, the above-mentioned ophthalmic preparation may further include at least one of the following additional technical features:

According to an embodiment of the present disclosure, the active ingredient is the salt of the compound represented by Formula (I), and the salt is a hydrochloride salt.

According to an embodiment of the present disclosure, the ophthalmic preparation is for use in treatment or prevention of myopia and/or amblyopia.

According to embodiments of the present disclosure, the myopia and/or amblyopia is caused by one or more selected from myopic shift in diopter, myopic vitreous cavity depth extension, myopic axial length extension, visual form deprivation, anisometropia, and astigmatism.

According to an embodiment of the present disclosure, the myopia and/or amblyopia includes one or more of mild myopia, moderate myopia, high myopia, axial myopia, simple myopia, pathological myopia, distance vision loss, asthenopia, exotropia, strabismus amblyopia, fundus damage, visual occlusion, visual distortion, double vision, abnormal color vision, abnormal light vision, decreased contrast sensitivity, and ametropic amblyopia. It should be noted that the vision-impairing eye diseases described herein do not include vision problems caused by trauma.

In a fourth aspect of the present disclosure, the present disclosure provides a method for treating or preventing myopia and/or amblyopia. According to an embodiment of the present disclosure, the method includes: administering the above-described composition or the above-described ophthalmic preparation to an ocular surface of a subject. The Applicant has found through a number of experiments that the above-mentioned composition or ophthalmic preparation can be used to delay the progression of myopia in the subject, having a therapeutic effect on myopia, and amblyopia of the subject can be also substantially recovered to normal levels.

According to an embodiment of the present disclosure, the method is for use in the treatment and/or prevention of myopia and/or amblyopia.

According to an embodiment of the present disclosure, the myopia and/or amblyopia is caused by one or more selected from myopic shift in diopter, myopic vitreous cavity depth extension, myopic axial length extension, visual form deprivation, anisometropia, and astigmatism.

According to an embodiment of the present disclosure, the myopia and/or amblyopia includes one or more of mild myopia, moderate myopia, high myopia, axial myopia, refractive myopia, simple myopia, pathological myopia, distance vision loss, asthenopia, exotropia, strabismus amblyopia, ocular axis elongation, fundus damage, visual occlusion, visual distortion, double vision, abnormal color vision, abnormal light vision, decreased contrast sensitivity, anisometropic amblyopia, ametropic amblyopia, unilateral visual form deprivation amblyopia, or bilateral visual form deprivation amblyopia.

Additional aspects and advantages of the present disclosure will be set forth in part in the description which follows and, in part, will be apparent from the description, or may be learned by practice of the present disclosure.

### BRIEF DESCRIPTION OF THE DRAWINGS

FIG. 1 shows a change of relative crystalline lens thickness in respective groups before and after the administration (Mean ± SD, n = 6 eyes/group);
FIG. 2 shows a change of relative pupil diameter in respective groups before and after the administration (Mean ± SD, n = 6 eyes/group).

### DETAILLED DESCRIPTION

Hereinafter, embodiments of the present disclosure will be described in detail. The embodiments described below are exemplary and illustrative only and are not to be construed as limiting the present disclosure. Where specific techniques or conditions are not specified in the embodiments, they are performed according to techniques or conditions described in the literature in the art or according to the product description. The reagents or instruments used are conventional products that can be obtained commercially without indicating the manufacturer.

Furthermore, the terms "first" and "second" are used for descriptive purposes only and shall not be construed as indicating or implying relative importance or implicitly indicating the number of technical features indicated. Thus, a feature defined with "first" or "second" may explicitly or implicitly comprise at least one such feature. In the present disclosure, the meaning of "plurality" is at least two, for example, two, three, etc., unless clearly and specifically limited otherwise.

### Pharmaceutical use

The uses of the compound having the structure represented by Formula (I), or the nitrogen oxide, solvate, metabolite, pharmaceutically acceptable salt or prodrug thereof, according to the present disclosure, in the preparation of the medicament include: the preparation of a medicament for treating, preventing, ameliorating, controlling or alleviating the vision-impairing diseases in mammals, especially humans, and the preparation of other medicaments with insignificant toxic side effects for inhibiting M receptors.

The term "solvate" in the present disclosure indicate that the compound has a solvent on surface, in crystal lattice, or both on the surface and in the crystal lattice. A specific example of solvate is a hydrate, in which the solvent on the surface, in the crystal lattice, or both is water. Hydrates may or may not have other solvents than water on the surface, in the crystal lattice, or both.

The term "metabolite" in the present disclosure refers to a product of metabolism of the compound having the structure represented by Formula (I) or the salt thereof in the body. Metabolites of the compound can be identified by known techniques, and the activity thereof can be characterized by assay as described herein. As used herein, the "pharmaceutically acceptable salts" refer to organic and inorganic salts of the compounds according to the present disclosure.

As used herein, the term "prodrug" refers to a compound that can be converted in vivo to the compound represented by Formula (I). Such a conversion is affected by hydrolysis of the prodrug in blood or by enzymatic conversion of the prodrug to the parent structure in blood or tissue.

### Pharmaceutical compositions

As used herein, the term "composition" refers to a product comprising the specified ingredients in the specified amounts, as well as any product that is directly or indirectly resulted from combination of the specified ingredients in the specified amounts. The term associated with the pharmaceutical composition is intended to encompass the products containing one or more active ingredients, and one or more inert ingredients that make up the carrier, as well as any products that are directly or indirectly resulted from combination, complexation or aggregation of any two or more of the ingredients, or from dissociation of one or more of the ingredients, or from other types of reactions or interactions of one or more of the ingredients. Accordingly, the pharmaceutical composition according to the present disclosure encompasses any composition prepared by mixing the compound according to the present disclosure and a pharmaceutically acceptable carrier.

The pharmaceutical composition provided by the present disclosure includes at least one pharmaceutically acceptable carrier. For example, a formulation of the pharmaceutical composition is an ophthalmic preparation such as eye drops, eye ointment, eye cream, eye emulsion, eye gel, eye pills, eye film, intraocular implant.

The pharmaceutical composition provided by the present disclosure may further includes carriers such as suitable antimicrobial agents or preservatives, suitable isotonic agents, suitable antioxidants, suitable local anesthetics, suitable suspending and dispersing agents, suitable emulsifying agents, suitable sequestering agents or chelating agents, suitable pH-adjusting agents, etc.

The pharmaceutical composition according to the present disclosure can be used in veterinary treatment of mammals such as pets, introduced species, and farm animals, in addition to being beneficial for vision-impairing eye diseases of human beings. Examples of additional animals include horses, dogs, and cats. The compound according to the present disclosure includes pharmaceutically acceptable derivatives thereof.

The present disclosure is further described with reference to specific embodiments, which are merely for illustration rather than limiting the present disclosure.

It should be understood that the data results in the following examples are all presented in Mean ± SD.

### Examples: Preparation of Penehyclidine Hydrochloride Solution, as Samples for Test

Penehyclidine hydrochloride and normal saline were mixed and stirred until completely dissolved, to prepare 0.01%, 0.1%, 0.5%, 1% and 2% penehyclidine hydrochloride solutions as samples for test, respectively.

### Effect Examples

### Effect Example 1: Effects of Penehyclidine Hydrochloride on Crystalline Lens and Pupils of Monkeys

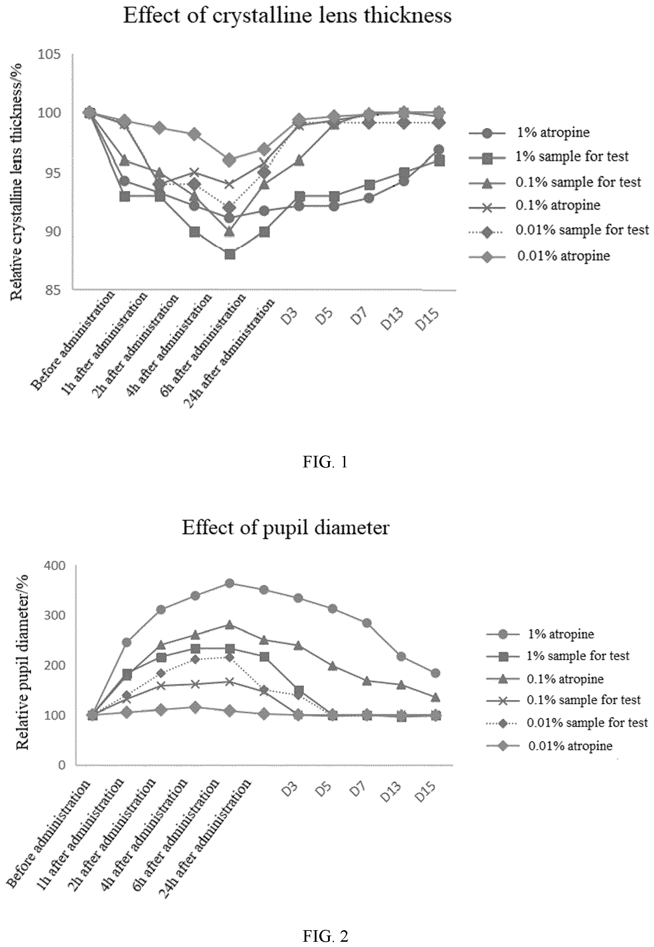

Healthy Cynomolgus monkeys qualified for yearly quarantine inspection, aged 2.5 to 3.5 years, weighing 2.0 to 4.0 kg, male or female, were selected and randomly divided into 6 groups, 3 monkeys in each group, with a total of 18 monkeys. The first, second, and third groups were administrated with 1%, 0.1% and 0.01% atropine sulfate eye drops, respectively. The 1%, 0.1% and 0.01% atropine sulfate eye drops were prepared by diluting the commercially available 1% atropine sulfate eye drops with PBS to different concentrations. The fourth, fifth, and sixth groups were administrated with 1%, 0.1% and 0.01% penehyclidine hydrochloride solutions, for both eyes each time, 30 µL/eye. Before the administration and at 1h, 2h, 4h, 6h, 24h, D3, D5, D7, D13 and D15 after the administration, the crystalline lens thickness and pupil diameter of both eyes were examined using A-ultrasound, and the relative crystalline lens thickness and pupil diameter (Mean ± SD, n = 6 eyes/group) were calculated by taking the data before the administration as criteria. The results are shown in FIG. 1 and FIG. 2.

The results of lens thickness are shown in FIG. 1. At 6h after the administration, the crystalline lens thickness of each group was decreased to the minimum. The higher the concentration, the stronger the effect of reducing the crystalline lens thickness. The effects of reducing lens thickness at 6h after the administration are ranked in such an order of 1% sample for test> 0.1% sample for test ≈ 1% atropine> 0.01% sample for test> 0.1% atropine> 0.01% atropine. Each group was gradually recovered from D1. The lower the concentration, the faster the recovery. The groups administrated with the samples for test with the concentrations from 0.01% to 0.1% and the atropine group were completely recovered in 3 to 5 days after the administration, and the group administrated with the sample for test with the concentration 1% and the group administrated with 1% atropine did not completely recover in 14 days after the administration. Penehyclidine hydrochloride has a definite dose-related relaxation effect on the ciliary muscle in vivo, which can delay the progression of myopia and achieve the treatment of myopia, and it has stronger effect than atropine.

Pupil diameter results are shown in FIG. 2. The group administrated with 0.01% sample for test did not produce mydriasis. Pupil diameter in other groups showed dose-related expansion, reaching the maximum value at 6h after the administration. The effects of mydriasis are ranked in such an order of 1% atropine> 0.1% atropine> 1% sample for test> 0.01% atropine> 0.1% sample for test. After 6h, the subjects were slowly recovered from mydriasis. The groups administrated with 0.1% to 1% samples for test and the group administrated with 0.01% atropine were recovered to the normal 2 to 4 days after the administration (D3-D5), and the group administrated with 0.1% to 1% atropine were not completely recovered 14 days after the administration (D15). Compared with the atropine groups, the mydriatic side effects in the group administrated with 0.1% to 1% samples for test were significantly reduced and the recovery time was shorter, indicating that the side effects of photophobia and blurred vision caused by pupil dilation can be significantly reduced.

### Effect Example 2: Treatment of Visual form deprivation Myopia in Guinea Pigs with penehyclidine hydrochloride

### Two-week-old weaned, healthy short-hair tricolor guinea pigs, male or female, were selected. After passing quarantine, 48 guinea pigs with monocular diopter of ≥ + 1.00D and binocular anisometropia of ≤ 1.50D were selected and randomly divided into 6 groups: groups administrated with samples for test (high, medium and low dose), negative control group, and positive control group (atropine group), with 8 animals in each group. All animals were modeled at the left eye, and the right eye was not treated. The modeling method was isoflurane inhalation anesthesia, and the nylon fastener bottom surface of the self-made styrene plastic diffuser was adhered to the skin around the eyes by using cyanoacrylate glue. The adhesion was confirmed to be firm. The diffuser could be freely disassembled and installed. There was no light leakage except the diffuser. The disinfection and surgical procedures were controlled within 3 to 5 min. The animals were awakened after 5 to 10 min. On the modeling day, 0.01%, 0.1%, 0.5% and 1% penehyclidine hydrochloride solution, 1% atropine sulfate eye drops and phosphate buffer solution (PBS) were administered to the conjunctival sac of modeled eyes, once a day, 20 µL/eye/time, continuously administrated for 6 weeks. The animal status and diffuser adhesion status were observed once daily during the test. Diopter and ocular axis length were measured respectively before the test (D-1), two weeks after the test (D14), four weeks after the test (D28) and six weeks after the test (D42) by using retinoscopy and A-ultrasound. The results are shown in Table 1 and Table 2.

**[Table 1] Results of diopter**

| Group | | Control eyes-OD | | | | Modeled eyes-OS | | | |
|---|---|---|---|---|---|---|---|---|---|
| | | D-1 | 2 weeks | 4 weeks | 6 weeks | D-1 | 2 weeks | 4 weeks | 6 weeks |
| PBS | Mean ± SD | 5.25 ±1.95 | 4.69 ±1.83 | 4.75 ±1.85 | 4.75 ±2.00 | 5.13 ±1.79 | -2.69±3.21a | -4.00±3.34a | -5.06±1.04a |
| 1% atropine | Mean ± SD | 5.21 ±1.45 | 4.88 ±1.28 | 4.96 ±1.32 | 4.83 ±1.37 | 5.00 ±1.64 | 0.03±2.25ab | 0.89±2.47ab | 1.1 8:1:2. 52ab |
| 1% sample for test | Mean ± SD | 5.05 ±1.05 | 4.85 ±1.06 | 4.98 ±1.12 | 4.85 ±1.05 | 5.08 ±1.14 | - 0.05±1.80ab | - 0.08±1.45ab | - 0.28±1.79ab |
| 0.5% sample for test | Mean ± SD | 5.17 ±1.50 | 4.88 ±1.38 | 4.96 ±1.29 | 4.88 ±1.32 | 4.83 ±1.27 | - 0.03±2.91ab | - 0.13±1.93ab | - 0.35±1.56ab |
| 0.1% sample for test | Mean ± SD | 4.83 ±1.80 | 4.81 ±2.05 | 4.78 ±1.49 | 4.88 ±1.51 | 4.81 ±1.62 | - 0.08±2.57ab | - 0.16±2.26ab | - 0.48±1.49ab |
| 0.01% sample for test | Mean ± SD | 4.72 ±1.39 | 4.72 ±1.30 | 4.61 ±1.32 | 4.61 ±1.29 | 4.72 ±1.60 | - 0.09±3.18ab | - 0.63±2.76ab | - 1.06±2.48ab |

| | | | | | | | | | |
|---|---|---|---|---|---|---|---|---|---|
| Note: a indicates p ≤ 0.05% compared to D-1; b indicates p ≤ 0.05% compared to concurrent PBS group. | | | | | | | | | |

The diopter results are shown in Table 1. Compared with D-1, the diopter values of the modeled eyes at different time periods were statistically different. 2 weeks after the administration, the modeled eyes of animals in the group administrated with PBS eye drops induced relative myopia of -2.69 ± 3.21D, and the diopter of each dose group shifted toward myopia, but no relative myopia was induced. The relative myopia degree of modeled eyes in the PBS group increased with time, while that in other groups increased slightly. After administration for 6 weeks, relative myopia of -5.06 ± 1.04D was induced in the modeled eyes of the animals in the group administrated with PBS eye drops. The group administrated with 1% atropine eye drops and the groups administrated with 1%, 0.5%, 0.1% and 0.01% penehyclidine hydrochloride solutions were induced with relative myopia of -1.18 ± 2.52D, -0.28 ± 1.79D, - 0.35 ± 1.56D, -0.48 ± 1.49D, and -1.06 ± 2.48D, respectively. The diopter values of each group were significantly lower than that of PBS group (P <0.05%). The results indicate that each treatment group had a significant effect on the treatment of myopia, and the groups administrated with sample for test at different concentrations had lower relative myopia degrees, and has stronger effect on the treatment of myopia than the group administrated with 1% atropine.

**[Table 2] Result of ocular axis elongation**

| | Ocular axis elongation with respect to control eyes (OD) (mm) | | | Ocular axis elongation in modeled eyes (OS) (mm) | | |
|---|---|---|---|---|---|---|
| | 2 weeks | 4 weeks | 6 weeks | 2 weeks | 4 weeks | 6 weeks |
| PBS | 0.36 ±0.22 | 0.50 ±0.26 | 0.72 ±0.22 | 0.52 ±0.13a | 0.68 ±0.11a | 0.94 ±0.12a |
| 1% atropine | 0.29 ±0.14 | 0.43 ±0.21 | 0.72 ±0.17 | 0.48 ±0.19a | 0.64 ±0.21a | 0.82 ±0.15 |
| 1% sample for test | 0.31 ±0.12 | 0.45 ±0.11 | 0.69 ±0.14 | 0.46 ±0.12a | 0.50 ±0.13b | 0.66 ±0.16b |
| 0.5% sample for test | 0.25 ±0.11 | 0.42 ±0.11 | 0.63 ±0.15 | 0.47 ±0.15a | 0.48 ±0.12b | 0.76 ±0.15b |
| 0.1% sample for test | 0.26 ±0.15 | 0.42 ±0.15 | 0.65 ±0.20 | 0.41 ±0.19a | 0.46 ±0.16b | 0.70 ±0.16b |
| 0.01% sample for test | 0.37 ±0.15 | 0.53 ±0.16 | 0.80 ±0.23 | 0.50 ±0.10a | 0.56 ±0.15 | 0.82 ±0.15 |

| | | | | | | |
|---|---|---|---|---|---|---|
| Note: a indicates p <0.05% compared to OD over the same period; b indicates p <0.05% compared to PBS. | | | | | | |

The ocular axis length of guinea pigs at D-1 was 8.2 to 8.3mm, and there was no significant difference in the ocular axis elongation between left and right eyes in each group (P> 0.05%) and between groups (P> 0.05%). The results of ocular axis elongation are shown in Table 2. Two weeks after the administration, the ocular axis elongation of modeled eyes was significantly greater than that of OD eyes (P <0.05%), indicating that modeling caused significant axial length growth in addition to normal ocular axis elongation.

Four weeks after the administration, the ocular axis elongation of OS eyes in PBS group and 1% atropine group was still significantly greater than that of OD eyes in the same period (P <0.05%), and there was no significant difference between the groups administrated with 0.01% to 1% samples for test and OD eyes in the same period (P> 0.05%). Ocular axis elongation in OS eyes in the groups administrated with 1.0, 0.5%, and 0.1% samples for test was significantly smaller than that in OS eyes in PBS group (P <0.05%), indicating that the groups administrated with samples for test at respective concentrations significantly delayed the ocular axis elongation earlier than that in atropine group.

Six weeks after the administration, the ocular axis elongation of OS eyes in the PBS group was still significantly greater than that in OD eyes in the same period (P <0.05%). There was no significant difference in ocular axis elongation of OS eyes in each dose group and OD eyes in the same period (P> 0.05%). Axial grow in OS eyes in the groups administrated with 1.0, 0.5%, and 0.1% samples for test was significantly smaller than OS eyes in the PBS group (P <0.05%). The ocular axis elongation of the OS eyes in the group administrated with 0.01% sample for test and the ocular axis elongation of the group administrated with 1% atropine were not significantly different from that of the OS eyes in the PBS group (P> 0.05%), indicating that each dose group had the effect of delaying the ocular axis elongation, and the ocular axis elongation in the groups administrated with 0.1% to 1% samples for test was smaller, indicating the stronger effect of treating myopia than that of 1% atropine.

In conclusion, the 0.01%~l% penehyclidine hydrochloride solution provided by the embodiments of the present disclosure can effectively treat the progression of myopia, and is more effective than 1% atropine sulfate eye drop.

### Effect Example 3: Investigation on Penehyclidine Hydrochloride in Treatment of Amblyopia of Cats

Twenty-four common 4-week-old domestic cats were randomly divided into a PBS group, a test group administrated with 1% penehyclidine hydrochloride solution, a test group administrated with 2% penehyclidine hydrochloride solution, and a control group administrated with 1% atropine, with 6 cats in each group. The right lateral rectus muscle of the cats in each group was cut off at 4 weeks of age. After the formation of amblyopia was determined by pattern visual evoked potential (P-VEP) test at 4 weeks after surgery, phosphate buffer solution (PBS), 1% and 2% samples for test, 1% atropine sulfate eye drops were respectively administered through conjunctival sac eye drops once a day, 20 µL/eye/time for 12 weeks successively. The P-VEP was recorded every 4 weeks. The results are shown in Table 3.

**[Table 3] P-VEP Results**

| Time | Group | PBS group | 1% sample for test | 2% sample for test | 1% atropine |
|---|---|---|---|---|---|
| Before modeling | Latency (ms) | 48.65 ±2.3 | 48.43 ±3.4 | 48.58 ±2.5 | 48.60 ±4.0 |
| | Amplitude (nv) | 55.65 ±3.2 | 55.58 ±3.5 | 55.78 ±2.9 | 55.46 ±3.5 |
| 4 weeks after modeling (Pre-treatment) | Latency (ms) | 58.23 ±4.5 | 58.45 ±5.2 | 58.32 ±5.0 | 58.15 ±4.9 |
| | Amplitude (nv) | 38.24 ±2.9 | 38.12 ±1.8 | 38.05 ±2.1 | 39.07 ±1.9 |
| 4 weeks after treatment | Latency (ms) | 59.75 ±4.1 | 57.33 ±4.3 | 56.39 ±2.5 | 57.67 ±2.3 |
| | Amplitude (nv) | 32.34 ±2.8 | 42.72 ±3.6 | 43.98 ±3.6 | 41.79 ±2.9 |
| 8 weeks after treatment | Latency (ms) | 62.39 ±3.4 | 55.26 ±4.5 | 54.45 ±3.2 | 56.58 ±3.2 |
| | Amplitude (nv) | 27.55 ±2.9 | 48.72 ±3.6 | 50.54 ±3.4 | 47.65 ±2.7 |
| 12 weeks after treatment | Latency (ms) | 64.23 ±3.2 | 51.03 ±4.3 | 50.25 ±3.5 | 54.36 ±4.6 |
| | Amplitude (nv) | 20.34 ±2.8 | 52.55 ±3.1 | 53.67 ±3.1 | 50.78 ±3.8 |

As shown in Table 3, the latency and amplitude (nv) of P wave in each group were basically the same before modeling. After 4 weeks of modeling, the latency of P-VEP in each group was significantly prolonged and the amplitude was significantly reduced, which were statistically significant compared with those before modeling, indicating that the animal model of amblyopia was successfully created in each group after 4 weeks of right lateral rectus amputation. The latency of P-VEP in the PBS group continued to extend and the amplitude continued to decrease during continuous administration for 4 to 12 weeks. The P-VEP trend in each treatment group was gradually recovered, the latency was gradually shortened, and the amplitude was gradually increased. Twelve weeks after the administration, the groups administrated with 1% and 2% samples for test were almost recovered to pre-modeling, and the amblyopia recovery effect of the groups administrated with 1% and 2% samples for test was higher than that of the group administrated with 1% atropine. The results indicate that 1% to 2% penehyclidine hydrochloride solutions had better effect on amblyopia than atropine.

### Effect Example 4: Investigation on Repeated Eye Drop Toxicity of Penehyclidine Hydrochloride in Rabbits

30 healthy Dutch rabbits in total, male or female, qualified after quarantine, were selected and randomly divided into 5 groups, 6 rabbits in each group, including a solvent control group, a control group administrated with 1% atropine, test groups respectively administrated with 0.01%, 1% and 2% penehyclidine hydrochloride solutions. The animals were administered with eye drops in bilateral conjunctival sac once a day, 30 µL/eye/time, repeated for 12 weeks. All animals were observed for death, morbidity, respiration, secretions, feces, diet and drinking at least once daily during the test. Each animal was visually observed for pupillary light reflex prior to each administration, and eye irritation was observed within 1 minute after each administration, including, but not limited to, squinting, blinking and head flick. If the animal is found with any of these abnormalities, the frequency (counts per minute) and/or duration (recorded as shorter than or longer than 60 seconds) of occurrence is recorded. Prior to administration in D1, 4-th week, 8-th week and 12-th week, all animals received local observation on eyes, including, but not limited to, redness, swelling, conjunctival hyperemia and secretion. The anterior segment and fundus were examined by using hand-held slit lamp and direct ophthalmoscope. The results are shown in Table 4 to Table 6.

**[Table 4] Eye Irritation Results**

| | Squinting | | Blinking | | Throwing head | | Reflection of light in pupils | Status |
|---|---|---|---|---|---|---|---|---|
| Group | Frequency | Duration | Frequency | Duration | Frequency | Duration | | |
| Solvent control group | √ | √ | √ | √ | √ | √ | √ | √ |
| 1% atropine group | 13 | L | 16 | L | √ | √ | Disappear | √ |
| Group administrated with 2% sample for test | 3 | S | 4 | S | √ | √ | Decrease | √ |
| Group administrated with 1% sample for test | √ | √ | √ | √ | √ | √ | √ | √ |
| Group administrated with 0.01% sample for test | √ | √ | √ | √ | √ | √ | √ | √ |

| | | | | | | | | |
|---|---|---|---|---|---|---|---|---|
| Note: frequency means times per minute, S means duration shorter than 60s, L means duration longer than 60s, √ means no abnormality. | | | | | | | | |

As shown in Table 4, the eye irritation results indicate that 1% atropine group showed moderate irritation, and the reflection to light by pupils disappeared. Except the slight irritation and reduced reflection to light in the pupils in 2% high-dose group, the groups administrated with 1% and 0.01% samples for test exhibited no irritation, and similar to the solvent control group, they had normal reflection to light in the pupils, indicating that penehyclidine hydrochloride is safe in ocular administration and had insignificant effects on pupils.

**[Table 5] Ophthalmic Examination Results**

| Time | | Solvent control group | 1% atropine group | Group administrated with 2% sample for test | Group administrated with 1% sample for test | Group administrated with 0.01% sample for test |
|---|---|---|---|---|---|---|
| D1 | Corneal opacity | 0 | 0 | 0 | 0 | 0 |
| | Conjunctival chemosis | 0 | 0 | 0 | 0 | 0 |
| | Intraocular pressure | 17 | 25 | 18 | 16 | 17 |
| 4-th week | Corneal opacity | 0 | 1 | 0 | 0 | 0 |
| | Conjunctival chemosis | 0 | 0 | 0 | 0 | 0 |
| | Intraocular pressure | 18 | 23 | 16 | 17 | 17 |
| 8-th week | Corneal opacity | 0 | 1 | 0 | 0 | 0 |
| | Conjunctival chemosis | 0 | 1 | 0 | 0 | 0 |
| | Intraocular pressure | 16 | 26 | 17 | 16 | 18 |
| 12-th week | Corneal opacity | 0 | 1 | 0 | 0 | 0 |
| | Conjunctival chemosis | 0 | 1 | 0 | 0 | 0 |
| | Intraocular pressure | 18 | 24 | 18 | 17 | 17 |

[Table 6]

**Results of ocular local observations**

| Corneal opacity (% range) | | Conjunctival chemosis and swelling | |
|---|---|---|---|
| 0 | Cornea was normal without turbidity | 0 | Normal, no edema |
| 1 | 1% to 25% area of visible substrate turbidity | 1 | Slight edema, no eyelid eversion |
| 2 | 26% to 50% area of visible substrate turbidity | 2 | Moderate edema resulting in incomplete upper and lower eyelid closure; edema was confined to the upper eyelid, which was partially everted |
| 3 | 51% to 75% area of visible substrate turbidity | 3 | Obvious edema accompanied with partial eversion of the upper and lower eyelids, position of which were easily determined by frontal observation and observation of the eyelid position |
| 4 | 76% to 100% area of visible substrate turbidity | 4 | Significant upper and lower eyelid eversion, upper eyelid more obvious than lower eyelid, difficult to recover |

As shown in Table 6, after repeated administration of samples for test at different concentrations for 12 weeks, no corneal opacity and conjunctival chemosis were observed, and intraocular pressure was similar to the solvent control group. However, 4 weeks after the repeated administration, slight corneal opacity and slight conjunctival edema were observed in the atropine group, indicating that the safety of repeated administration of penehyclidine hydrochloride was significantly superior to that of atropine at the same concentration.

In the specification, references to descriptions of the terms "an embodiment", "some embodiments", "examples", "specific examples", or "some examples", etc. mean that a particular feature, structure, material, or characteristic described in connection with the embodiment or example is included in at least one embodiment or example of the present disclosure. In this specification, schematic representations of the terms are not necessarily directed to the same embodiment or example. Furthermore, the particular features, structures, materials, or characteristics described may be combined in any suitable manner in any one or more embodiments or examples. Furthermore, combinations and compositions of the various embodiments or examples and features of the various embodiments or examples described in this specification can be made by those skilled in the art without contradiction among each other.

The embodiments of the present disclosure illustrated and described above are merely exemplary and not intend to limit the present disclosure. Those skilled in the art can make changes, modifications, substitutions and alterations to the above embodiments without departing from the scope of the present disclosure.

## Claims

1. Use of a compound having a structure represented by Formula (I), or a nitrogen oxide, solvate, metabolite, pharmaceutically acceptable salt or prodrug thereof, in the preparation of medicament for treating and/or preventing vision-impairing eye diseases,

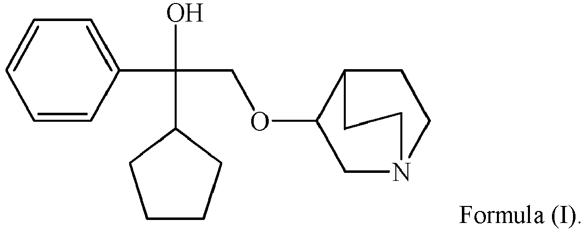

2. The use according to claim 1, **characterized in that**, the salt of the compound represented by Formula (I) is selected from organic salts or inorganic salts.

3. The use according to claim 1, **characterized in that**, the salt of the compound represented by Formula (I) is a hydrochloride salt, and preferably, the hydrochloride salt has a structure represented by Formula (II),

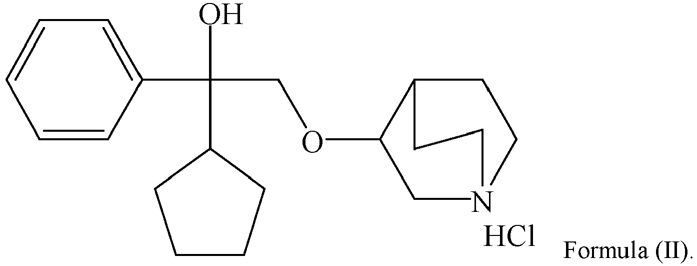

4. The use according to claim 1, **characterized in that**, the medicament is for use in the treatment and/or prevention of myopia and/or amblyopia.

5. The use according to claim 4, **characterized in that**, the myopia and/or amblyopia is caused by one or more selected from myopic shift in diopter, myopic vitreous cavity depth extension, myopic axial length extension, visual form deprivation, anisometropia, and astigmatism.

6. The use according to claim 1, **characterized in that**, the medicament is for use in the treatment and/or prevention of myopia and/or amblyopia, the myopia and/or amblyopia comprising one or more of mild myopia, moderate myopia, high myopia, axial myopia, refractive myopia, simple myopia, pathological myopia, distance vision loss, asthenopia, exotropia, strabismus amblyopia, ocular axis elongation, fundus damage, visual occlusion, visual distortion, double vision, abnormal color vision, abnormal light vision, decreased contrast sensitivity, anisometropic amblyopia, ametropic amblyopia, unilateral visual form deprivation amblyopia, or bilateral visual form deprivation amblyopia.

7. A pharmaceutical composition for treating and/or preventing vision-impairing eye diseases, **characterized in that**, the pharmaceutical composition comprises, as an active ingredient, the compound having the structure represented by Formula (I), or the nitrogen oxide, solvate, metabolite, pharmaceutically acceptable salt or prodrug thereof according to claim 1.

8. The pharmaceutical composition according to claim 7, **characterized in that**, the pharmaceutical composition further comprises at least one pharmaceutically acceptable carrier.

9. The pharmaceutical composition according to claim 7, **characterized in that**, the pharmaceutical composition is in a formulation of an ophthalmic preparation, the ophthalmic preparation comprising one or more of eye drops, eye ointment, eye cream, eye emulsion, eye gel, eye pills, eye film, or intraocular implant.

10. The pharmaceutical composition according to claim 7, **characterized in that**, a mass fraction of the active ingredient in the pharmaceutical composition ranges from 0.005% to 2%, based on a mass of the pharmaceutical composition.

11. The pharmaceutical composition according to any one of claims 7 to 10, **characterized in that**, the pharmaceutical composition is for use in the treatment or prevention of myopia and/or amblyopia.

12. The pharmaceutical composition according to claim 11, **characterized in that**, the myopia and/or amblyopia is caused by one or more selected from myopic shift in diopter, myopic vitreous cavity depth extension, myopic axial length extension, visual form deprivation, anisometropia, and astigmatism.

13. The pharmaceutical composition according to claim 11, **characterized in that**, the myopia and/or amblyopia comprises one or more of mild myopia, moderate myopia, high myopia, axial myopia, refractive myopia, simple myopia, pathological myopia, distance vision loss, asthenopia, exotropia, strabismus amblyopia, ocular axis elongation, fundus damage, visual occlusion, visual distortion, double vision, abnormal color vision, abnormal light vision, decreased contrast sensitivity, anisometropic amblyopia, ametropic amblyopia, unilateral visual form deprivation amblyopia, or bilateral visual form deprivation amblyopia.

14. An ophthalmic preparation for treating and/or preventing vision-impairing eye diseases, **characterized in that**, a mass fraction of an active ingredient in the ophthalmic preparation ranges from 0.005% to 2%, and the active ingredient is the compound having the structure represented by Formula (I), or the nitrogen oxide, solvate, metabolite, pharmaceutically acceptable salt or prodrug thereof according to claim 1.

15. The ophthalmic preparation according to claim 14, **characterized in that**, the active ingredient is a salt of the compound represented by Formula (I), and the salt is a hydrochloride salt.

16. The ophthalmic preparation according to any one of claims 14 to 15, **characterized in that**, the ophthalmic preparation is for use in the treatment or prevention of myopia and/or amblyopia.

17. The ophthalmic preparation according to claim 16, **characterized in that**, the myopia and/or amblyopia is caused by one or more selected from myopic shift in diopter, myopic vitreous cavity depth extension, myopic axial length extension, visual form deprivation, anisometropia, and astigmatism.

18. The ophthalmic preparation according to claim 16, **characterized in that**, the myopia and/or amblyopia comprises one or more of mild myopia, moderate myopia, high myopia, axial myopia, refractive myopia, simple myopia, pathological myopia, distance vision loss, asthenopia, exotropia, strabismus amblyopia, ocular axis elongation, fundus damage, visual occlusion, visual distortion, double vision, abnormal color vision, abnormal light vision, decreased contrast sensitivity, anisometropic amblyopia, ametropic amblyopia, unilateral visual form deprivation amblyopia, or bilateral visual form deprivation amblyopia.

19. A method for treating and/or preventing vision-impairing eye diseases, **characterized in that**, the method comprises:
administering the composition according to any one of claims 7 to 13 or the ophthalmic preparation according to any one of claims 14 to 18 to an ocular surface of a subject.

20. The method according to claim 19, **characterized in that**, the method is for use in the treatment and/or prevention of myopia and/or amblyopia.

21. The method according to claim 20, **characterized in that**, the myopia and/or amblyopia is caused by one or more selected from myopic shift in diopter, myopic vitreous cavity depth extension, myopic axial length extension, visual form deprivation, anisometropia, and astigmatism.

22. The method according to claim 20, **characterized in that**, the myopia and/or amblyopia comprises one or more of mild myopia, moderate myopia, high myopia, axial myopia, refractive myopia, simple myopia, pathological myopia, distance vision loss, asthenopia, exotropia, strabismus amblyopia, ocular axis elongation, fundus damage, visual occlusion, visual distortion, double vision, abnormal color vision, abnormal light vision, decreased contrast sensitivity, anisometropic amblyopia, ametropic amblyopia, unilateral visual form deprivation amblyopia, or bilateral visual form deprivation amblyopia.
